(19) Europäisches Patentamt
European Patent Office
Office européen des brevets

(11) **EP 4 757 064 A1**

(12) **EUROPEAN PATENT APPLICATION**

(43) Date of publication:
**10.06.2026 Bulletin 2026/24**

(21) Application number: **25315296.1**

(22) Date of filing: **06.08.2025**

(51) International Patent Classification (IPC):
*H01P 1/18* (2006.01)    *H03H 7/18* (2006.01)
*H03H 7/20* (2006.01)    *A61B 5/00* (2006.01)
*G16H 50/50* (2018.01)    *G01R 33/563* (2006.01)

(52) Cooperative Patent Classification (CPC):
**G16H 50/50; A61B 5/4094; H03H 7/18; H03H 7/20;**
**G01R 33/56341**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH LA MA MD TN**

(30) Priority: **09.12.2024 EP 24315559**

(71) Applicant: **STMicroelectronics International N.V.**
**1228 Plan-les-Ouates, Geneva (CH)**

(72) Inventors:
• **Azevedo De Souza E Vecchia, Vinicius**
  **38240 MEYLAN (FR)**
• **Knopik, Vincent**
  **38830 CRETS EN BELLEDONNE (FR)**

(74) Representative: **Cabinet Beaumont**
  **4, Place Robert Schuman**
  **B.P. 1529**
  **38025 Grenoble Cedex 1 (FR)**

(54) **REFLECTION-TYPE PHASE SHIFTER DEVICE**

(57)    The present disclosure relates to a reflection-type phase shifter device (700), RTPS, comprising at least two reflective loads (102, 104) whose values can be set independently of one another.

## Fig. 7

## EP 4 757 064 A1

### Description

<u>Technical field</u>

The present disclosure relates generally to reflection-type phase shifter (RTPS) devices and their control methods.

<u>Background art</u>

RTPS devices are phase shifters based on a 90° coupler and a variable reflective load. When varying the capacitance presented by the reflective load, the phase shift value changes.

One of the biggest challenges with RTPS devices is that the function between the value of the charge capacitance and the phase shift observed is highly non-linear and frequency dependent.

<u>Summary of Invention</u>

There is a need to provide RTPS devices with an improved available phase range, i.e. with an improved phase shift response accuracy within a small variation in insertion loss.

One embodiment addresses all or some of the drawbacks of known RTPS devices.

One embodiment provides a reflection-type phase shifter device, RTPS, comprising at least two reflective loads whose values can be set independently of one another.

One embodiment provides a method for controlling a reflection-type phase shifter device, RTPS, comprising independent setting of respective values of at least two reflective loads.

According to one embodiment, each of the reflective loads is configured to have a respective configurable capacitance value.

According to one embodiment, each of the reflective loads comprises several branches configured to be coupled in parallel with each other, each of the branches comprising at least one capacitance and at least one switch coupled in series.

According to one embodiment, the switches are bipolar transistors.

According to one embodiment, each of the reflective loads comprises six branches and the value of the capacitance of each of the reflective loads is set according to a respective code, each of the bits of said code being associated with a control state of one of the switches of the respective reflective load.

According to one embodiment, the device further comprises a 90° coupler, two ports of which are coupled to the reflective loads.

According to one embodiment, the coupler is a hybrid 90° coupler.

According to one embodiment, the device further comprises a control unit configured to implement, in a calibration step, an algorithm for selecting the values of the reflective loads.

According to one embodiment, said algorithm comprises at least some of the following steps:

a) selecting a centre frequency;
b) determining, for each codes, the corresponding insertion losses and phase shifting obtained;
c) selecting a range of insertion loss variation values;
d) determining a first set of codes leading to insertion loss values which are included in the selected range of insertion loss variation values selected in step c); and
e) selecting, from said first set of codes determined in step d), a second set of codes wherein each code leads to a phase shift that is the closest to a different given phase shift value.

According to one embodiment, said algorithm further comprises at least some of the following steps:

e) computing an error value, for example of the RMS type, for each phase shift associated with each of the codes of the second set ;
f) comparing each of the previously computed error values with an authorized value or a range of error value;
g) when all or a given number of the previously computed error values are equal to or fall within the authorized error value or values, completing the reflective load value selection algorithm, or, when none, or a given number, of the previously calculated error values are equal to or within the authorized error value(s), repeating steps c) to g) by selecting a wider range of insertion loss variation values than previously.

According to one embodiment, the centre frequency value is equal to 10.7 GHz, or 11.7 GHz, or 12.6 GHz, or 14.5 GHz.

According to one embodiment, at the end of the calibration step, a look up table is obtained wherein phase shift targets are linked to respective code values to apply to the reflective loads.

According to one embodiment, the control unit is configured to set the respective value of said reflective loads according to said calibration step results.

According to one embodiment, the control unit is configured to set the values of each of the reflective loads based on said look up table.

Brief description of drawings

The foregoing features and advantages, as well as others, will be described in detail in the following description of specific embodiments given by way of illustration and not limitation with reference to the accompanying drawings, in which:

Figure 1 illustrates an example of an RTPS device;
Figure 2 illustrates an example of a 90° coupler of the RTPS device of Figure 1;
Figure 3 illustrates an example of a 90° coupler of the RTPS device of Figure 1;
Figure 4 illustrates an example of a 90° coupler of the RTPS device of Figure 1;
Figures 5a, 5b, 5c, 5d, 5e, illustrate examples of a variable reflective load of the RTPS device of Figure 1;
Figure 6 illustrates phase vs a control code of a variable reflective load of the RTPS device of Figure 1;
Figure 7 illustrates an example of an RTPS device according to an embodiment;
Figure 8 illustrates an example of a calibration method of an RTPS device according to an embodiment; and
Figure 9 illustrates an example of several steps of the control method of the example of Figure 8.

Description of embodiments

Like features have been designated by like references in the various figures. In particular, the structural and/or functional features that are common among the various embodiments may have the same references and may dispose identical structural, dimensional and material properties.

For the sake of clarity, only the operations and elements that are useful for an understanding of the embodiments described herein have been illustrated and described in detail.

Unless indicated otherwise, when reference is made to two elements connected together, this signifies a direct connection without any intermediate elements other than conductors, and when reference is made to two elements coupled together, this signifies that these two elements can be connected, or they can be coupled via one or more other elements.

In the following disclosure, unless indicated otherwise, when reference is made to absolute positional qualifiers, such as the terms "front", "back", "top", "bottom", "left", "right", etc., or to relative positional qualifiers, such as the terms "above", "below", "higher", "lower", etc., or to qualifiers of orientation, such as "horizontal", "vertical", etc., reference is made to the orientation shown in the figures.

Unless specified otherwise, the expressions "around", "approximately", "substantially" and "in the order of" signify within 10 % or 10°, and preferably within 5 % or 5°.

In the text, the RTPS devices disclosed are devices related to frequencies ranging for example from 1 to 100 GHz, for example 10.7 GHz, 12.6 GHz, or 14.5 GHz.

Phased arrays have been widely used in numerous millimeter-wave (mm-Wave) wireless systems to perform beam forming and spatial filtering that can enhance the equivalent isotropically radiated power for the transmitter, suppress the interferences and increase signal-to-noise ratio (SNR) for the receiver. The applications of mm-Wave phased array systems include ultra-high data-rate transmission (e.g., WirelessHD), 5G-6G communication, radar and imaging systems. Phase shifters (PS) play a key role in a phased array system, since it governs the beam forming quality and steering capabilities. A high-performance phase shifter should achieve a low insertion loss (IL), a wide phase shifting range, dense phase shift angles, and good input/output matching accuracy. Reflection-type phase shifters (RTPS) exhibit several unique advantages over the other passive phase shifter topologies, including continuous and dense phase shift, moderate size, and cascadable operation. Therefore, RTPS is an excellent candidate to meet the stringent requirements in high performance phased array systems.

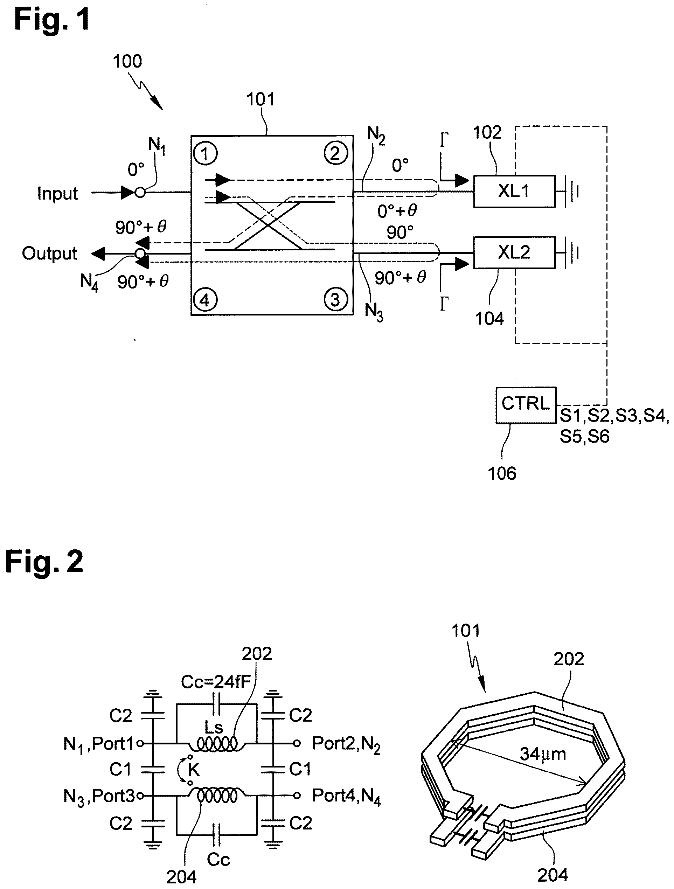

Figure 1 illustrates an example of an RTPS device 100.

The RTPS device 100 of Figure 1 comprises for example a 90° coupler 101 having a signal input node N1, an output node (also called isolation port) N4, a node N2 (also called through port), and a node N3 (also called couple port).

Nodes N2 and N3 are respectively coupled to a variable impedance, otherwise said a variable (or tuneable) capacitance, reflective load 102, 104. The reflective loads 102, 104 are for example identical or similar. The reflective loads 102, 104 are configured to reflect signals instead of absorbing it. In an example, the reflective loads 102, 104 have their capacitance value controlled according to at least one signal or several signals S1, S2, S3, S4, S5, S6, generated by a control unit 106 (CTRL). The signals S1, S2, S3, S4, S5, S6 are in this example common to both reflective loads 102, 104. In an example, the control unit 106 is comprised in the RTPS device 100. In another example, the control unit 106 is implemented outside of the RTPS device 100.

In an example, the reflective loads 102, 104 are coupled to ground.

In an example, the tuneable reflective loads 102, 104 topologies comprise tuneable, or variable, capacitors, series L-C resonators, parallel L-C resonators, C-L-C n-networks, or transformer-based reflective loads. The impedance of each of the reflective load is tuned to achieve the desired phase shifting angle and range. The variable capacitances 102, 104, which act as reflective loads, impact mostly the phase range, phase error and gain error. Different types of tuneable reflective capacitances can be found in the following reference: IEEE TRANSACTIONS ON CIRCUITS AND SYSTEMS-I: REGULAR PAPERS, VOL. 65, NO. 4, APRIL 2018.

The phase shift $\Theta$ is given by the phase of a reflection coefficient $\Gamma$ of the impedance (or loads) $Z_L$ of the reflective loads 102, 104.

The device 100 exhibits insertion losses IL, which are related to a magnitude of the reflection coefficient. Insertion losses are given by $IL=|Z_L-Z_0|/|Z_L+Z_0|$, where $Z_0$ is the characteristic impedance of the coupler 101.

In the represented example, the input signal is split into two paths with 90° phase difference by the 90° coupler. Next, the resulting two split signals are reflected from the two variable capacitances 102, 104 at the through and couple ports N2, N3. Assume that these two reflective loads are identical, the two reflected signals will be combined in-phase at the RTPS output (the isolation port N4) to generate the desired output phase shift.

In the device 100, the radiofrequency signal is fed at the input port N1 and recovered at isolation port, or the other way around, in a bidirectional manner.

The 90° coupler 101 impacts mostly the device 100 size, losses and the bandwidth. In an example, the 90° coupler 101 is designed with distributed or lumped elements.

Figure 2 illustrates an example of the 90° coupler 101 of the RTPS device of Figure 1. The coupler 101 is for example based on a balun coupler. In the represented example, the coupler 101 comprises two inductances 202, 204 with a coupling factor K. Inductance 202 couples nodes N1 and N2, and inductance 204 couples nodes N3 and N4. Similar capacitances of value C1 couple nodes N1 and N3, and nodes N2 and N4. Similar capacitances of value C2 couple respectively node N1 to ground, and node N2 to ground. Similar capacitances of value C2 couple respectively node N3 to ground, and node N4 to ground. Similar capacitances Cc couple respectively nodes N1 and N2, and N3 and N4.

In the represented example, the two inductances 202, 204 are formed by two superimposed heptagonal loops, for example with a 34 $\mu$m diameter for a 60 GHz central frequency.

Additional information about this type of coupler can be found in the reference entitled: 60GHz Low-Loss Compact Phase Shifters Using A Transformer-Based Hybrid in 65nm CMOS, 2011, M. Tabesh et al.

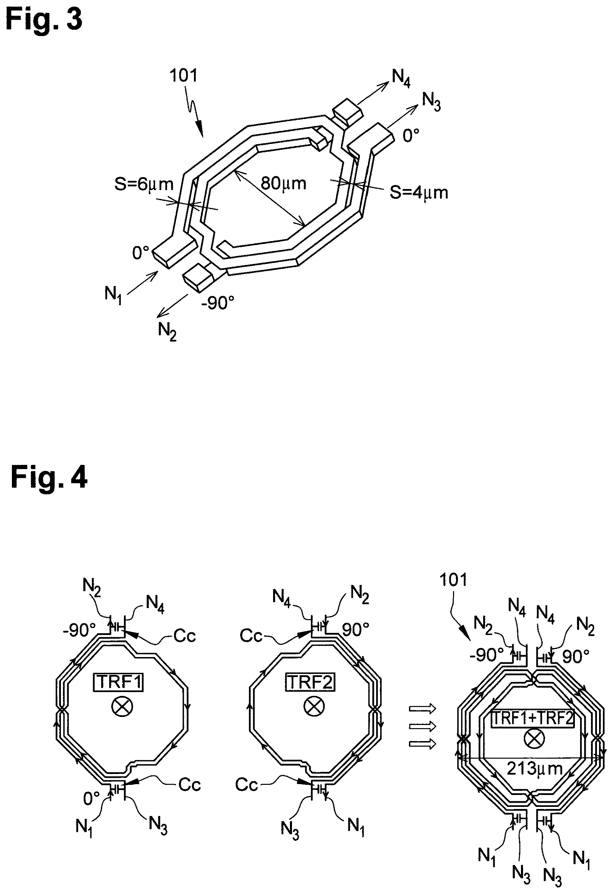

Figure 3 illustrates an example of the 90° coupler 101 of the RTPS device of Figure 1.

The coupler represented in Figure 3 is an example of a coupler with dimensions compatible with a frequency of 60 GHz. The coupler of Figure 3 has the same equivalent circuit as the coupler of Figure 2 but without the capacitances Cc. The coupler of Figure 3 comprises two interlaced loops arranged on two levels. A width W of the loops are for example of 6 $\mu$m, interloop spacing is of about 4 $\mu$m and a diameter of 80 $\mu$m. In this example, the nodes N1 and N2 are placed on a same side of the loops, and nodes N3 and N4 are arranged in the same metal level as the nodes N1 and N2, but placed diametrically apart from nodes N1 and N2.

Figure 4 illustrates an example of a 90° coupler 101 of the RTPS device of Figure 1.

The coupler 101 of Figure 4 is formed with two interlaced loops TRF1 and TRF2 which are globally mirror opposites. Each of these loops has two ends N2, N4 on the top of the loop in the orientation of the Figure 4, and two other ends N1, N3 oriented toward the bottom. Nodes N1 and N3 of each loop are coupled with a capacitance Cc, and nodes N2 and N4 of each loop are coupled with a capacitance Cc.

The nodes N1 of the two loops form a differential signal with a phase of 180°. The nodes N4 of the two loops form a differential signal with a phase of 180°. The two nodes N3 of the two loops are single ended nodes with a phase shift of 180° (between 0° and 180°). The nodes N2 of the two loops form a differential signal with a phase of 180° (between -90° and 90°).

The coupler 101 of Figure 4 forms an 8-port folded transformer-based quadrature coupler to generate fully differential I/Q signals within one inductor footprint of 213 $\mu$m for a 15 GHz frequency for example. Further information related to the type of coupler as in Figure 4 can be found in the following reference: IEEE TRANSACTIONS ON MICROWAVE THEORY AND TECHNIQUES, VOL. 63, NO. 12, DECEMBER 2015.

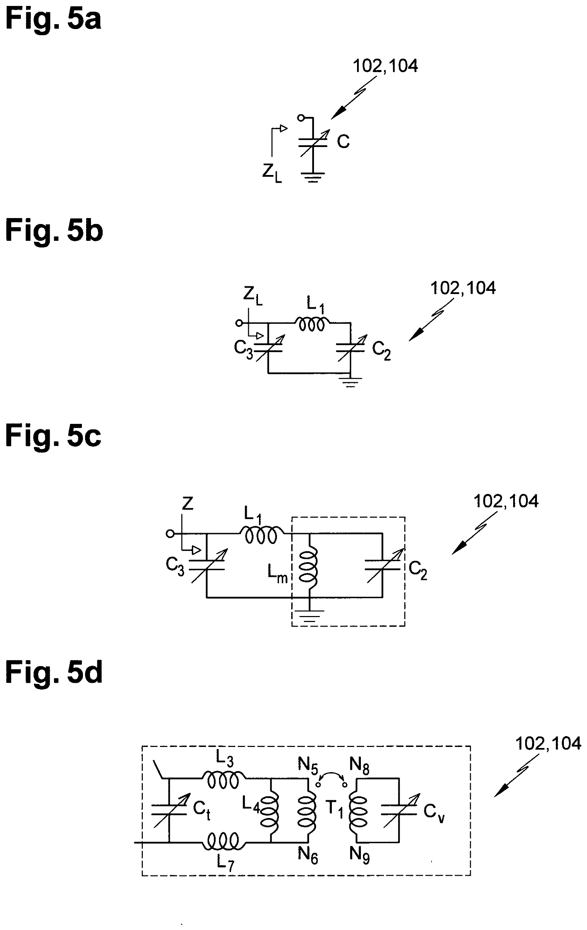

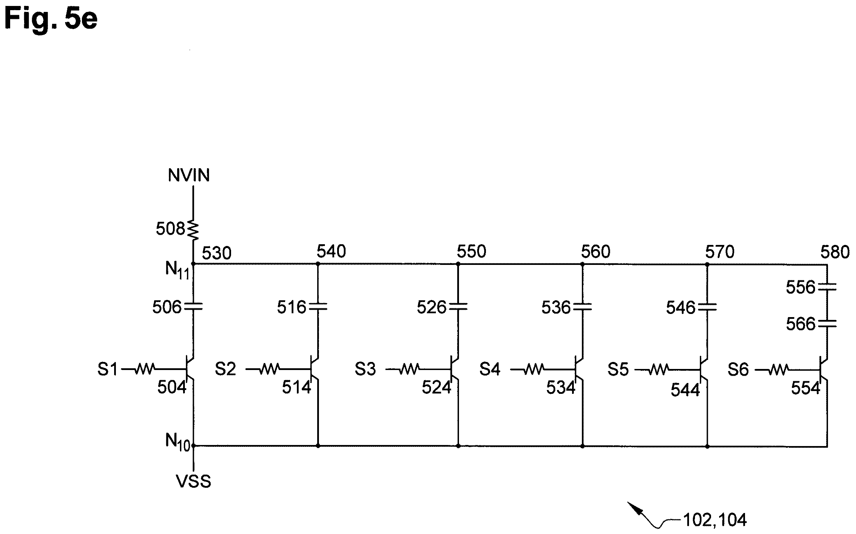

Figures 5a, 5b, 5c, 5d, 5e, illustrate examples of a variable reflective load of the RTPS device of Figure 1.

In the example of Figure 5a, each of the variable, or tuneable, reflective load 102, 104 is formed with a varactor or a capacitive switch bank.

The example of Figure 5a exhibits very limited phase excursion. In an example of an ideal variable capacitor tuning range of four (4 bits), it leads to a maximal 73.4° phase shift range.

In the example of Figure 5b, each of the variable load 102, 104 comprises a tuneable capacitor C3 coupling the respective node N2 or N3 to ground, and an impedance L1 in series with a tuneable capacitor C2 between the respective node N2 or N3 and ground.

The example of Figure 5b has a structure called CLC n-resonator. It allows a 360° phase excursion but with highly non-linear operating regions exhibiting large phase error.

In the example of Figure 5c, each of the variable load 102, 104 is similar to the example of Figure 5c except that an additional inductance Lm couples the middle point of inductance L1 and tuneable capacitor C2 to ground.

The tuneable reflective load of Figure 5c is based on a transformer formed by inductances L1 and Lm.

In the example of Figure 5d, each of the reflective load 102, 104 comprises a tuneable capacitor Ct coupling for example differential nodes N2 and N4 of a differential 90° coupler. The represented example comprises an inductance L3 coupling a first electrode of the capacitor Ct and a node N5 of a primary side of a transformer T1. An inductor L4 couples the node N5 of the primary side of the transformer and a node N6 of the primary side of the transformer. Additionally, an inductor L7 couples the node N6 to a second electrode of the tuneable capacitor Ct. In the represented example, a tuneable capacitor Cv couples two nodes N8, N9 of the secondary side of the transformer.

For a 62 GHz frequency, the example of Figure 5d provides a 367° phase shift and insertion losses between 3.7 and 10.3 dB.

In the example of Figure 5e, each of the variable reflective load 102, 104 has a configurable, or tuneable, capacitance (in an equivalent manner its impedance) value.

In the represented example, each of the reflective loads 102, 104 are structurally identical or similar, and for example comprises, each, six branches 530, 540, 550, 560, 570, 580 coupled, preferably connected, in parallel between a node N10 and a node N11. Each of the branches 530, 540, 550, 560, 570, 580 comprises at least one capacitance, respectively 506, 516, 526, 536, 546, 556, in series with at least one switch, respectively 504, 514, 524, 534, 544, 554.

In an example, the capacitance of each branch has an electrode coupled to a node N11 and the corresponding switch has a pin coupled to the node N10 which is coupled to a rail of application of a VSS potential (or ground). In another example, the capacitance of each branch has an electrode coupled to node N10 and the corresponding switch has a pin coupled to node N11.

In an example, the switches 504, 514, 524, 534, 544, 554, are bipolar or MOS type transistors.

Each switch 504, 514, 524, 534, 544, 554 of each reflective loads 102, 104 have a dedicated control signal respectively S1, S2, S3, S4, S5, S6. In the case where the switches are transistors, the control signals S1, S2, S3, S4, S5, S6 are applied, for example via a control resistance, to the gate or base of the respective transistor 504, 514, 524, 534, 544, 554.

The number of parallel branches of the reflective loads 102,104 can be less than six or more than six and be similar or different to the branches 530, 540, 550, 560, 570, 580.

In an example, the different branches are similar or identical within fabrication differences.

In an example, the capacitances 506, 516, 526, 536, 546, 556, have a similar or equal value.

In another example, the capacitances 506, 516, 526, 536, 546, 556, have different values between each other, for example to obtain a variation of the total capacitance of each of the reflective load 102, 104 to be exponential.

In the represented example, the branch 580 comprises two capacitors 556, 566 in series, but one single capacitor could also be envisaged.

In the represented example, an optional inductance 508 couples node N11 and a node NVIN. This inductance helps to improve the phase shift range.

In an example, node N11 of the reflective load 102 is coupled to node N2, and node N11 of the reflective load 104 is coupled to node N3.

In an example, node NVIN of the reflective load 102 is coupled to node N2, and node VIN of the reflective load 104 is coupled to node N4.

The value of the capacitance of each of the reflective loads 102, 104 is set according to a respective 6-bits code which is the number of switches. Each of the bits is associated with a control state of one of the switches 504, 514, 524, 534, 544, 554, of the respective reflective load 102, 104. In an example, a bit at 0 corresponds to a non-passing state of the switch, and a bit at 1 corresponds to a passing state of the corresponding switch. With 6-bits reflective loads, it leads to up to 64 phase shift possibilities.

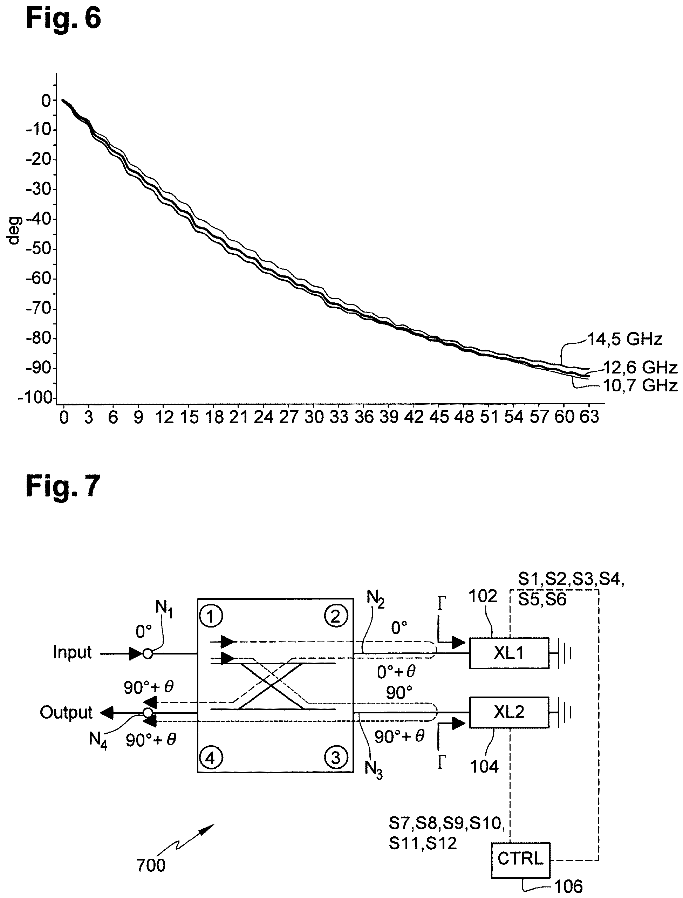

Figure 6 illustrates phase shift (deg) as a function of a control code of a variable reflective load 102, 104 of the RTPS device of Figure 1. More particularly, Figure 6 illustrates phase shift values (deg) depending on the 64 different bit codes (from 0 to 63) possible with a 6-bit tuneable reflective loads 102, 104 as the example of Figure 5e. In the example of Figure 6, the two reflective loads 102, 104 are similar and controlled with the same signals S1, S2, S3, S4, S5, S6, and the frequencies applied are 12.6 GHz, 10.7 GHz and 14.5 GHz.

In the represented example, one can see that the phase shift varies over a 90° tuning range (from 0 at code 0 down to -90° at a code 63) but does not vary linearly with the different bits controls of the capacitive reflective loads 102, 104. In a non-represented manner, it can be demonstrated that the maximum gain variation across all phase settings for this example is 0.6 dB. When considering the root mean square (RMS) error as compared to a linear response, the nonlinearity of the phase shift induces high RMS values.

In order to lower the phase error different solutions could be envisaged.

A first solution could be the use of larger bits capacitive reflective loads 102, 104. For example, having 12 bits capacitive loads 102, 104 instead of 6 bits capacitive loads. Thus, up to 64*64=4096 different settings could be achieved instead of 64. A subset of these settings, which minimize the error, could be chosen. This subset would be formed for example of 7 bits, i.e. 128 settings. Nevertheless, this solution is not desirable since larger bits would mean, in this case, adding capacitor bits in parallel, which in turn results in increased losses.

A second solution could be the use of more complex loads, capable of a large tuning range, as for example the CLC architecture described in the Figure 5b, and which allows a 360° phase shift for example, and re-tune it to a smaller phase range (for example 90°).

The proposed embodiments provide a Reflection-type phase shifter device, RTPS, comprising at least two reflective loads whose values can be set independently of one another.

It allows to double the setting possibilities (from 6-bits to 12-bits for example) since the two reflective loads whose values can be set independently. Moreover, in this case, there is no additional losses as compared to the first solution.

It moreover allows to improve the RMS phase shift error to be as close as possible from a linear relationship.

It also provides a phase tuning range above 120°.

Figure 7 illustrates an example of an RTPS device 700 according to an embodiment.

The RTPS device 700 of Figure 7 is similar to the RTPS device 100 of Figure 1 except that the switches 504, 514, 524, 534, 544, 554 of the tuneable capacitive load 104 are controlled with respective signals S7, S8, S9, S10, S11, S12 which may be different from the signals S1, S2, S3, S4, S5, S6 controlling respectively the switches 504, 514, 524, 534, 544, 554 of the tuneable capacitive load 102. Otherwise said, in the represented example, the control unit 106 is configured to control differently the capacitance of the load 102 and the capacitance of the load 104.

In the case of 6-bit capacitive loads 102, 104, it leads to a 12-bit phase shift setting possibility. Each phase shift corresponds to a bit code corresponding to the signal state applied to each of the twelve switches. Hence, 4096 phase shift settings are possible in this case. In an example, the first 6 bits of the code are related to the state of the signal applied to the respective switches of the reflective load 102 and the 6 last bits of the code are related to the state of the signal applied to the respective switches of the reflective load 104.

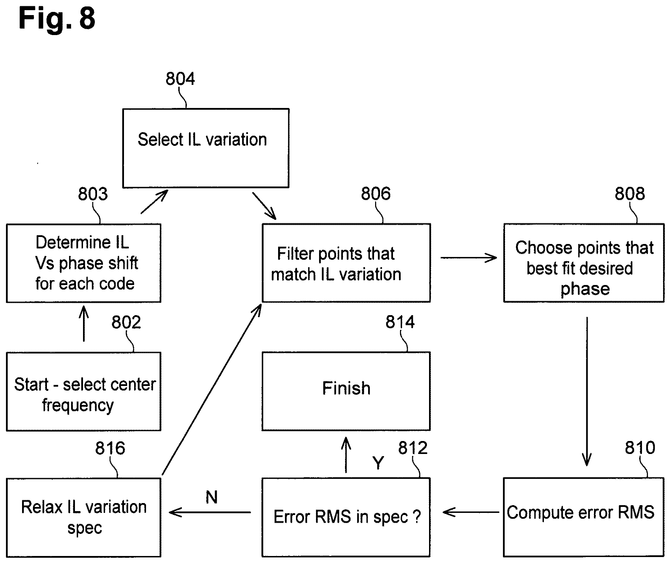

Figure 8 illustrates an example of a calibration method of the RTPS device 700 according to an embodiment. More particularly, the example calibration method of Figure 8 can be executed by a control unit, for example during a product characterization in factory and/or implemented autonomously in the field, for example during a self-test at each power-up.

In a step 802 (Start- select centre frequency), the central frequency of the input signal is selected for example with the control unit 106, for example 10.7 GHz, 12.6 GHz, 14.5 GHz or 62 GHz.

In a step 803 (Determine IL vs phase shift for each code), a first table is determined. The first table comprises for example, for each possible code (4096 points in the case of 2*6-bits) leading to a different reflective load capacitance value, an insertion loss value for a corresponding phase shift value. The first table can be determined numerically or experimentally. The step 803 can be implemented prior to step 802 or after step 802. In this example, the term "table" means a group of related data (code, phase shift, insertion losses insertion loss variation) which are for example stored in a memory of the RTPS device 100 or of the control unit 106 for example.

In a step 804 (Select IL variation), a range of insertion loss variation values or insertion loss values, for example expressed in dB, is determined or selected. Step 804 can be implemented prior or posterior to step 802 and/or step 803.

In a step 806 (Filter points that match IL variation), posterior to step 804, the codes of the first table, which lead to insertion losses falling into the selected range determined in step 804, are filtered and selected to be part of a first set of codes. The first set of codes is for example stored in a memory of the RTPS device 100 or of the control unit 106 for example.

In a step 808 (Choose points that best fit desired phase), posterior to step 806, a second set of codes is determined among the codes of the first set of codes. In step 808, the corresponding phase shift of each code of the first set is compared to a set of given (ideal phase) phase shift values. The given phase shift values are for example an incremental list of phase shift values starting for example from 0 up to 90° or 120° and incrementing with a given phase shift step, for example a 1.42° step. A code from the first set is selected to be in the second set when its corresponding phase shift value is the closest to one of the given phase shift values. For example, if the code 454 is part of the first set and if it corresponds to a phase shift value of for example 4.21°, then, if 4.21° is the closest, among the different phase shift values of the other codes of the first set, to a given phase shift value of 4.28 (4.28° resulting from an example of an incrementation of a given phase shift step of 1.42°), thus code 454 would be selected to be part of the second set. The second set of codes is for example stored in a memory of the RTPS device 100 or of the control unit 106 for example. The second set of codes is for example comprised in a look up table.

In other words, during step 808, the best points are chosen from a phase shift/IL constellation that performs the desired phase shift with acceptable insertion loss and/or insertion loss variation.

In a step 810 (Compute error RMS), posterior to step 808, an error value, for example of the RMS type, is calculated for each phase shift associated with each of the codes of the second set. In an example, the calculated error takes into account the errors $e_n$ of all settings, for example via the formula: $\sqrt{(e_0{}^2 + ... + e_n{}^2)/N}$ , $e_n$ being the error between ideal phase and real phase of code "n", and N being the number of codes. Other types of errors can be implemented based for example on average value or absolute error value.

For example, the code 454 corresponds to a phase shift of 4.21° which exhibits an error of 0.07 as compared to the ideal 4.28° obtained by a linear incrementation.

In a step 812 (Error RMS in spec?), posterior to step 810, each of the errors computed in step 810 are for example compared to a limit error range or to a single desired RMS error. In the case of the comparison to a limit error range, if all, or a given percentage, of the codes of the second set are associated with a phase shift error within the limit range (otherwise called an authorized value range), then (branch Y) a step 814 (Finish) is implemented, and the algorithm stops. If none, or a given percentage or a given number, of the codes of the second set are associated with a phase shift error outside the limit range, then (branch N) a step 816 (Relax IL variation spec) is implemented.

In step 816, the range of insertion loss variation, or insertion loss, values determined or selected in step 804 is widened and step 806 is implemented again. It allows to accept more entries in the first set of codes.

After step 814, the second set of codes contains codes values which can be used to build a look up table to link (within the error range) a phase shift target and the associated code value to apply to the reflective loads 102, 104.

At the end of the calibration method exposed in Figure 8, the end result is a look-up table which is used later on during normal operation of the device, to translate desired phases (in degrees) to control signals to activate the corresponding capacitors of the loads.

The control unit 506 can thus, using the obtained look up table, process the signals S1, S2, S3, S4, S5, S6, S7, S8, S9, S10, S11, S12 to apply, during normal operation of the device, a corresponding code value to the reflective loads 102, 104 in order to obtain a targeted phase shift (within the error range).

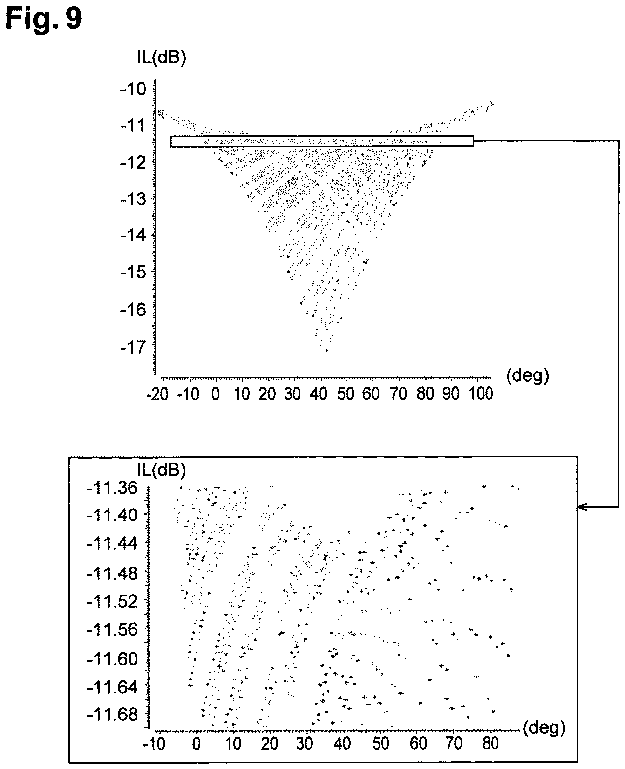

Figure 9 illustrates an example of several steps of the control method of the example of Figure 8. More particularly, Figure 9 represents, on the left graph, a constellation of points corresponding to the first table as determined in step 803 in the case of 2*6 bits for a 12.6 GHz central frequency. For each possible code (4096 codes in the case of 2*6 bits), an insertion loss value is reported as a function of a corresponding phase shift value.

Figure 9 illustrates additionally, on the graph on the right, the insertion loss associated to the phase shift which corresponds to the codes selected to be in the first set in step 806. In the represented example, the selected range of insertion losses (defined in step 804) is between -11.36 dB and -11.7 dB, and the corresponding phase shift values are between -10° and 90°. This phase shift range of between -10° and 90° is for example chosen to contain points in all this range without too large gaps.

The points of the close-up graph of the right, which corresponds to the first set of codes as determined in step 806, are the processed to follow the steps 808, 810 and 812. An example result of these steps is given in the Table 1 below:

Table 1

| Phase ideal | Phase real | Error | Code |
|---|---|---|---|
| 0.00 | 0.00 | 0.00 | 4086.00 |
| 1.42 | 1.27 | 0.16 | 982.00 |
| 2.85 | 2.81 | 0.03 | 3558.00 |
| 4.27 | 4.21 | 0.06 | 454.00 |
| 5.69 | 5.56 | 0.13 | 2758.00 |
| 7.12 | 7.07 | 0.05 | 1687.00 |
| 8.54 | 8.36 | 0.18 | 1274.00 |
| 9.96 | 9.92 | 0.04 | 3747.00 |
| 11.38 | 11.32 | 0.06 | 890.00 |
| 12.81 | 12.64 | 0.17 | 1709.00 |
| 14.23 | 14.16 | 0.07 | 346.00 |
| 15.65 | 15.60 | 0.05 | 1626.00 |
| 17.08 | 16.89 | 0.18 | 1642.00 |
| 18.50 | 18.49 | 0.01 | 665.00 |
| ... | ... | ... | ... |
| 88.23 | 87.14 | 1.09 | 2100 |

(continued)

| Phase ideal | Phase real | Error | Code |
|---|---|---|---|
| 89.65 | 89.65 | 0.00 | 20 |

In Table 1, a first column (Phase ideal) comprises incremental steps of an ideal phase shift from 0° to 89.65°. The steps are for example here of approximatively 1.42°.

A second column (Phase real) of Table 1 comprises the phase shift corresponding to codes belonging to the second set of codes as selected during step 808.

A third column (Error) of Table 1 reports the comparison between the ideal phase and the obtained phase. It corresponds to the "$e_n$" terms as determined during step 810.

A fourth column reports the codes of the second set corresponding to their associated phase shift.

For example, in order to obtain a theoretical phase shift of 18.50° with device 700, the code with the decimal value 665, coded on 12 bits, is selected and applied to the tuneable reflective loads 102, 104 in a way that the 6 first bits of the 665 code are dedicated to the configuration of the reflective load 102, and the last 6 bits of the same 665 code are dedicated to the configuration of the reflective load 104. In other words, 665 is coded as 001010011001 in binary, and so the first 6 bits (S [12:7]) are 001010 and the last 6 bits (S[6:1]) are 011001, so that the two reflective loads are respectively controlled with 001010 and 011001, each bit corresponding to a state of a controlling signal applied on a respective switch or transistor of the loads.

In another example, in order to obtain a theoretical phase shift of 17.03° with device 700, the code with the hexadecimal value 1642, coded on 12 bits, is selected and applied to the tuneable reflective loads 102, 104 in a way that the 6 first bits of the 1642 code are dedicated to the configuration of the reflective load 102, and the last 6 bits of the same code 1642 are dedicated to the configuration of the reflective load 104.

With Table 1, a 90° phase shifter, with a 1.42° step, and centred at 12.6 GHz, is obtained.

This example gives an RMS error of 0.45° with a gain variation of 0.2 dB and a maximum error of 1.4°.

When keeping the same settings but for a signal centred on 10.7 GHz, the error RMS is 3.2° for a gain variation of 1 dB and a max error of 4.8°. When keeping the same settings but for a signal centred on 14.5 GHz, the error RMS is 4.2° for a gain variation of 1 dB and a max error of 6.3°.

In order to keep similar performances over the full band 10.7 GHz-14.5 GHz, it is possible to have different code selection for transmission and reception channels.

In another example, similar to the example of Table 1 but for a central frequency of 11.7 GHz instead of 12.6 GHz, the error RMS is 0.2° for a gain variation of 0.2 dB and a max error of 0.8°. When keeping the same settings but for a signal centred on 10.7 GHz, the error RMS is 1.5° for a gain variation of 0.7 dB and a max error of 0.7°. When keeping the same settings but for a signal centred on 12.7 GHz, the error RMS is 2.1° for a gain variation of 0.8 dB and a max error of 3.2°.

The examples presented in Figures 7 to 9 allow, thanks to the independent load (i.e. capacitance) tuning of the reflective loads, to extend the number of phase settings of an RTPS type phase shifter which leads to a better accuracy.

Various embodiments and variants have been described. Those skilled in the art will understand that certain features of these embodiments can be combined, and other variants will readily occur to those skilled in the art. In particular, if the examples of Figure 9 have been presented with similar loads as the one of Figure 5e, the person of the art can use its knowledge to implement other kind of reflective loads 102, 104, for example the ones from Figures 5a to 5d as long as they are configurable or tuneable in an independent manner. The person of the art can use its knowledge to implement other kinds of 90° couplers such as the one disclosed in Figures 2, 3 and 4, for example a hybrid coupler.

Finally, the practical implementation of the embodiments and variants described herein is within the capabilities of those skilled in the art based on the functional description provided hereinabove. In particular, concerning the architecture of the reflective loads 102, 104, the man of the art can use its knowledge to implement other architectures of reflective loads as long as they can vary their capacitance in a controllable manner and with different signals states from one load 102 to the other 104. The person of the art may also use the present disclosure with different frequencies between for example 1 and 100 GHz.

**Claims**

1.  Reflection-type phase shifter device (700), RTPS, comprising at least two reflective loads (102, 104) whose values can be set independently of one another.

2.  Method for controlling a reflection-type phase shifter device (700), RTPS, comprising independent setting of respective values of at least two reflective loads.

3. Device according to claim 1, or method according to claim 2, in which each of the reflective loads (102, 104) is configured to have a respective configurable capacitance value.

4. Device according to claim 1 or 3, or method according to claim 2 or 3, in which each of the reflective loads (102, 104) comprises several branches (530, 540, 550, 560, 570, 580) configured to be coupled in parallel with each other, each of the branches comprising at least one capacitance (506, 516, 526, 536, 546, 556) and at least one switch (504, 514, 524, 534, 544, 554) coupled in series.

5. Device or method according to claim 4, wherein the switches (504, 514, 524, 534, 544, 554) are bipolar transistors.

6. Device or method according to any of claims 4 or 5, wherein each of the reflective loads (102, 104) comprises six branches (530, 540, 550, 560, 570, 580) and the value of the capacitance of each of the reflective loads is set according to a respective code, each of the bits of said code being associated with a control state (S1, S2, S3, S4, S5, S6, S7, S8, S9, S10, S11, S12) of one of the switches (504, 514, 524, 534, 544, 554) of the respective reflective load (102, 104).

7. Device according to any of claims 1 or 3 to 6, or method according to any of claims 2 to 6, further comprising a 90° coupler (101), two ports of which are coupled to the reflective loads (102, 104).

8. Device or method according to claim 7, wherein the coupler (101) is a hybrid 90° coupler.

9. Device according to any of claims 1 or 3 to 8, or method according to any of claims 2 to 8, further comprising a control unit (106) configured to implement, in a calibration step, an algorithm for selecting the values of the reflective loads (102, 104).

10. Device or method according to claim 9 in its dependency to claim 6, wherein said algorithm comprises at least some of the following steps:

   a) selecting a center frequency (802);
   b) determining (803), for each codes, the corresponding insertion losses and phase shifting obtained;
   c) selecting (804) a range of insertion loss variation values;
   d) determining (806) a first set of codes leading to insertion loss values which are included in the selected range of insertion loss variation values selected in step c); and
   e) selecting (808), from said first set of codes determined in step d), a second set of codes wherein each code leads to a phase shift that is the closest to a different given phase shift value.

11. Device or method according to claim 10, wherein said algorithm further comprises at least some of the following steps:

   e) computing (810) an error value, for example of the RMS type, for each phase shift associated with each of the codes of the second set ;
   f) comparing each of the previously computed error values with an authorized value or a range of error value;
   g) when all or a given number of the previously computed error values are equal to or fall within the authorized error value or values, completing the reflective load value selection algorithm, or, when none, or a given number, of the previously calculated error values are equal to or within the authorized error value(s), repeating steps c) to g) by selecting a wider range of insertion loss variation values than previously.

12. Device or method according to claim 10 or 11, wherein the center frequency value is equal to 10.7 GHz, or 11.7 GHz, or 12.6 GHz, or 14.5 GHz.

13. Device or method according to any of claims 9 to 12, wherein at the end of the calibration step, a look up table is obtained wherein phase shift targets are linked to respective code values to apply to the reflective loads (102, 104).

14. Device or method according to any of claims 9 to 13, wherein the control unit is configured to set the respective value of said reflective loads (102, 104) according to said calibration step results.

15. Device or method according to any of claims 13, or 14 in its dependency to claim 13, wherein the control unit is configured to set the values of each of the reflective loads (102, 104) based on said look up table.

## Fig. 1

## Fig. 2

## Fig. 3

## Fig. 4

# Fig. 5a

102,104

$Z_L$

C

# Fig. 5b

$Z_L$   $L_1$

102,104

$C_3$   $C_2$

# Fig. 5c

Z   $L_1$

102,104

$C_3$   $L_m$   $C_2$

# Fig. 5d

102,104

$L_3$   $N_5$   $N_8$

$C_t$   $L_4$   $T_1$   $C_v$

$L_7$   $N_6$   $N_9$

# Fig. 5e

EP 4 757 064 A1

# Fig. 6

# Fig. 7

# Fig. 8

804
Select IL variation

803
Determine IL
Vs phase shift
for each code

806
Filter points that
match IL variation

808
Choose points that
best fit desired
phase

802
Start - select center
frequency

814
Finish

816
Relax IL variation
spec

N

812
Y
Error RMS in spec ?

810
Compute error RMS

# Fig. 9

# EUROPEAN SEARCH REPORT

**Application Number**

EP 25 31 5296

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VOISIN STEEVEN ET AL: "A 25-50 GHz Digitally Controlled Phase-Shifter", 2021 16TH EUROPEAN MICROWAVE INTEGRATED CIRCUITS CONFERENCE (EUMIC), EUROPEAN MICROWAVE ASSOCIATION (EUMA), 3 April 2022 (2022-04-03), pages 305-308, XP034130081, DOI: 10.23919/EUMIC50153.2022.9783836 [retrieved on 2022-06-01] | 1-9 | INV.<br>H01P1/18<br>H03H7/18<br>H03H7/20<br>A61B5/00<br>G16H50/50<br>G01R33/563 |
| A | * abstract *<br>* the whole document *<br>----- | 10-15 | |
| X | CN 108 463 948 A (PEREGRINE SEMICONDUCTOR CORP) 28 August 2018 (2018-08-28) | 1-9, 13-15 | |
| A | * abstract; figures 1-5A, 9, 15 *<br>----- | 10-12 | |
| X | FANG CHUNHUI ET AL: "Systematic Design of a Broadband Reflective-Type Phase Shifter with Minimal Loss Variation and High Phase Accuracy", 2022 IEEE 4TH INTERNATIONAL CONFERENCE ON CIRCUITS AND SYSTEMS (ICCS), IEEE, 23 September 2022 (2022-09-23), pages 144-148, XP034222428, DOI: 10.1109/ICCS56666.2022.9936288 [retrieved on 2022-11-07] | 1-9 | |
| A | * Abstract<br>I. Introduction<br>II. Circuit design;<br>page 144 - page 147; figures 1,2,4 *<br>----- | 10-15 | TECHNICAL FIELDS SEARCHED (IPC)<br><br>H01P<br>H03H |

-/--

The present search report has been drawn up for all claims

| Place of search | Date of completion of the search | Examiner |
|---|---|---|
| The Hague | 20 January 2026 | Pastor Jiménez, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

page 1 of 2

# EP 4 757 064 A1

**EUROPEAN SEARCH REPORT**

Europäisches Patentamt / European Patent Office / Office européen des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WANG HAO ET AL: "A 24.25-29.50 GHz 6-Bit Active Vector-Summing Phase Shifter with Low Gain Variation for 5G Communication", 2024 IEEE MTT-S INTERNATIONAL WIRELESS SYMPOSIUM (IWS), IEEE, 16 May 2024 (2024-05-16), pages 1-3, XP034734243, DOI: 10.1109/IWS61525.2024.10713808 [retrieved on 2024-10-24] | 1-9 | |
| A | * the whole document * | 10-15 | |
| A | US 2019/190147 A1 (HALLIVUORI JUHA SAMUEL [FI]) 20 June 2019 (2019-06-20) * paragraph [0005] * * paragraph [0042] - paragraph [0054]; figures 2-6 * | 1-15 | |

TECHNICAL FIELDS SEARCHED (IPC)

The present search report has been drawn up for all claims

| Place of search | Date of completion of the search | Examiner |
|---|---|---|
| The Hague | 20 January 2026 | Pastor Jiménez, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 25 31 5296

20-01-2026

| Patent document cited in search report | | Publication date | Patent family member(s) | | Publication date |
|---|---|---|---|---|---|
| CN 108463948 | A | 28-08-2018 | CN | 108463948 A | 28-08-2018 |
| | | | US | 2017194688 A1 | 06-07-2017 |
| | | | WO | 2017119948 A1 | 13-07-2017 |
| US 2019190147 | A1 | 20-06-2019 | CN | 111742504 A | 02-10-2020 |
| | | | EP | 3729694 A1 | 28-10-2020 |
| | | | US | 2019190147 A1 | 20-06-2019 |
| | | | WO | 2019122506 A1 | 27-06-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *IEEE TRANSACTIONS ON CIRCUITS AND SYSTEMS-I: REGULAR PAPERS*, April 2018, vol. 65 (4)
- **M. TABESH**. *60GHz Low-Loss Compact Phase Shifters Using A Transformer-Based Hybrid in 65nm CMOS*, 2011
- *IEEE TRANSACTIONS ON MICROWAVE THEORY AND TECHNIQUES,* December 2015, vol. 63 (12)